# EUROPÄISCHE PATENTSCHRIFT

(11) **EP 3 769 701 B1**
(45) Veröffentlichungstag und Bekanntmachung des Hinweises auf die Patenterteilung: **13.11.2024**
(21) Anmeldenummer: 20187697.6
(22) Anmeldetag: 24.07.2020
(51) Int. Cl.: A61B 17/14, A61B 17/00

(54) **VERBUNDSÄGEBLATT FÜR EINE MEDIZINISCHE KNOCHENSÄGE**
COMPOSITE SAW BLADE FOR A MEDICAL BONE SAW
LAME DE SCIE COMPOSITE POUR UNE SCIE À OS MÉDICALE

(30) Priorität: 26.07.2019 DE 102019120294
(43) Veröffentlichungstag der Anmeldung: 27.01.2021
(73) Patentinhaber: Aesculap AG, 78532 Tuttlingen (DE)
(72) Erfinder: HÖGERLE, Roland-Alois, 78532 Tuttlingen (DE); SCHAZ, Uwe, 78579 Neuhausen (DE); ZIERIS, Gerold, 78532 Tuttlingen-Möhringen (DE); MATTES, Uwe, 78532 Tuttlingen (DE); RASCHKA, Tim Dominik, 79110 Freiburg im Breisgau (DE); KELLER, Hans, 78589 Dürbheim (DE); WÄSCHLE, Tobias, 78598 Königsheim (DE)
(74) Vertreter: Winter, Brandl - Partnerschaft mbB

(56) Entgegenhaltungen:
- EP-A1- 1 607 058
- WO-A1-2016/142213
- DE-C1- 10 100 630
- DE-U1- 20 220 637
- US-A- 5 735 866
- US-A1- 2017 027 586

## Beschreibung

Die vorliegende Erfindung betrifft ein Sägeblatt für eine medizinische Knochensäge, insbesondere für eine Femur- und/oder Tibiaresektion, mit einem proximalen Haltebereich, welcher zur Kopplung an einer Sägevorrichtung dient, einem Führungsbereich mit zumindest einem Gleitelement, welches an einem biegesteife Sägeblattkern angeordnet ist und eine Kontaktfläche zur Führung des Sägeblatts in einem Führungsschlitz einer Sägeschablone bereitstellt, und einem distalen, Sägezähne aufweisenden Sägebereich.

### Stand der Technik

In der Knieendoprothetik werden vor einer Implantation einer Knieendoprothese der Femur- und Tibiaknochen eines Patienten präpariert, wofür diese gesägt werden müssen. Im Zuge dessen werden sogenannte Kniesägeschablonen oder Kniesägeblöcke, insbesondere aus metallischen Materialien, verwendet, welche über ein Ausrichtungssystem relativ zu dem Patienten präzise positioniert werden und zur Führung eines Sägeblattes dienen, um einen Sägeschnitt winkel- und positionsgenau ausführen zu können.

Eine bei derartigen Sägeanordnungen bestehende Problematik betrifft die Relativbewegung und die daraus resultierende Friktion zwischen dem Sägeblatt und der Sägeschablone (Reibpartner) während der Sägeschnitt vorgenommen wird. Genauer ausgedrückt, können nachfolgend beschriebene Probleme bei diesem Arbeitsschritt bisher nicht komplett vermieden werden. Durch die Reibung zwischen dem Sägeblatt und der Sägeschablone entsteht an den entsprechenden Komponenten Materialabrieb (Abriebpartikel), insbesondere Metallabrieb. Ferner führt die friktionsbedingte Oberflächenbelastung zu Korrosion an der Sägeschablone. Demgemäß unterliegen die Sägeschablonen einem hohen Verschleiß, was die Führungseigenschaften der Sägeschablone verschlechtert und zu ungenauen Sägeschnitten führen kann oder eine Repositionierung der Sägeschablone notwendig macht. An den Reibpartnern kommt es zudem zu einer erhöhten Wärmeentwicklung. Aufgrund dessen ist der Patient im Situs-Bereich einer erhöhten Belastung durch Wärmeeintrag bzw. Kontaminierung durch Abriebpartikel ausgesetzt. Die Patientensicherheit steht bei der Produktentwicklung in Zukunft immer mehr im Vordergrund. Aus diesem Grund sind die vorstehend aufgezeigten Probleme zu reduzieren oder bestenfalls gänzlich zu vermeiden.

Darüber hinaus kann es zwischen dem oszillierenden Sägeblatt und der/dem Sägeschablone/-block zu Kollisionen kommen. Dies führt zu einer erhöhten Geräuschemission während der Anwendung im OP. Des Weiteren ist eine Leichtbauweise von chirurgischen Instrumenten wie Sägevorrichtungen, insbesondere von chirurgischen, akkubetriebenen Handsägen erforderlich. Ein Hauptpunkt, welcher das Gewicht des chirurgischen Instrumentes definiert, ist die Masse eines in dem Instrument bereitgestellten Akkus. Die Masse des Akkus wird wiederum durch eine Anzahl der Zellen (Akkukapazität) bestimmt. Diese wird meist anhand der maximal zu bewegenden trägen Massen der Werkzeuge und deren zeitlicher Anwendung im OP vorgegeben. Das heißt, aufgrund der hohen schwingendenden Masse der Kniesägeblätter ist eine hohe Akkuleistung notwendig. Das Kniesägeblatt ist in der Regel das massenschwerste Werkzeug, welches an die Knochensäge adaptiert wird und gibt somit direkt die Dimensionierung des Akkus vor. Eine Reduzierung der schwingenden Masse am Sägeblatt wird daher indirekt die Masse des Akkus und der Gesamtmaschine nennenswert beeinflussen.

Der Stand der Technik beschreibt z.B. in WO 2016/142213 A1 ein Sägeblatt mit abstehenden keramischen Anlageelementen zur Minimierung des Abriebs an den Sägeblättern, welche in Verbindung mit zugehörigen Sägeblöcken aus Kunststoff mit keramischen Führungsflächen zur Sägeblattführung angewendet werden können. Dabei ist nicht bekannt, ob die abstehenden keramischen Anlageelemente des Sägeblatts stoffeinstückig mit dem Sägeblatt ausgebildet sind, oder ob diese daran fixiert sind. Das heißt, es ist nicht bekannt, wie eine Verbindung zwischen den abstehenden keramischen Anlageelementen und dem Sägeblatt gelöst ist. Darüber hinaus weisen derartige Sägeblätter mit abstehenden keramischen Anlageelemente folgende Probleme auf. Zum einen erreichen Keramiken meist keine endkonturnahe Maßhaltigkeit bei Herstellung, z.B. aufgrund des Verzugs der Keramik nach dem Sintern, weshalb eine Nachbearbeitung durch weitere Arbeitsschritte wie Schleifen, Polieren o.Ä. immer erforderlich ist. Keramiken wie beispielsweise Zirkoniumoxid oder Aluminiumnitrid sehr spröde und hart und entsprechend schwer zu verarbeiten. Demgemäß ist eine Herstellung eines solchen Sägeblatts teuer. Dies gilt gleichermaßen für einen damit zu verwendenden Sägeblock mit keramischen Führungselementen. Bei diesem besteht darüber hinaus die Gefahr, dass sich die Befestigung der Führungselemente am Sägeblock über die Zeit lockert. Ferner ist die Gefahr einer Beschädigung durch eine Kollision o.Ä. hoch. Bei Verwendung eines Sägeblatts mit harten Keramikelementen mit einer/m konventionellen Sägeschablone/ Sägeblock aus vergleichsweise weichem Stahl würde dieser hohem Verschleiß unterliegen und würde ein hoher, den Patienten belastender Metallabrieb entstehen.

Ferner ist aus EP 1 607 058 A1 bezieht sich auf ein chirurgisches Sägeblatt mit einem plattenförmigen Körper, welcher mit einem Einspannbereich und mit Sägezähnen versehen ist, wobei beidseitig an dem plattenförmigen Körper zumindest ein erhabener Bereich ausgebildet ist, der mit einer Sägeschablone in Anlage bringbar ist, wobei der erhabene Bereich durch Materialauftrag auf die Oberfläche des plattenförmigen Körpers gebildet ist.

US 2017/0027586 A1 offenbart ein sich hin- und herbewegendes chirurgisches Sägeblatt mit einem distalen Abschnitt, einem proximalen Abschnitt und einem Hauptkörper, der sich zwischen dem distalen Abschnitt und dem proximalen Abschnitt erstreckt. Der Hauptkörper umfasst eine obere Fläche und eine untere Fläche sowie eine erste Außenkante, die sich von der oberen Fläche zur unteren Fläche des Hauptkörpers erstreckt, und eine zweite Außenkante, die sich von der oberen Fläche zur unteren Fläche des Hauptkörpers erstreckt. Ein biokompatibles Polymer ist entlang mindestens einer der oberen Fläche, der unteren Fläche, der ersten Außenkante oder der zweiten Außenkante des Hauptkörpers vorgesehen.

### Zusammenfassung der Erfindung

Die der vorliegenden Erfindung zugrunde liegende Aufgabe besteht darin, Nachteile des Stands der Technik zu verbessern oder zu beseitigen. Insbesondere ist es eine Aufgabe der Erfindung, die Sägeanwendung bei der Femur- und Tibiaresektion zu verbessern, wobei in erster Linie die Sicherheit der Patienten und das OP-Ergebnis verbessert werden soll. Weiter insbesondere soll ein Sägeblatt bereitgestellt werden, welches leicht, einfach und kostengünstig zu fertigen ist und zudem mit einer gängigen Sägeschablone verwendbar ist, ohne dass übermäßige Abnutzung, Korrosion oder sonstige Schäden an den jeweiligen Komponenten entstehen und/oder ohne einen Patienten zu gefährden. Vorzugsweise soll ferner die Wärme- und/oder Geräuschentwicklung bei der Verwendung minimiert werden und/oder die benötigte Akkuleistung reduziert werden.

Die der Erfindung zugrunde liegende Aufgabe wird durch ein Sägeblatt mit den Merkmalen des Anspruchs 1 gelöst

Genauer ausgedrückt, wird zur Lösung der Aufgabe ein Sägeblatt für eine medizinische Knochensäge, insbesondere für eine Femur- und/oder Tibiaresektion, bereitgestellt, mit einem proximalen Haltebereich, welcher zur Kopplung an einer Sägevorrichtung dient, einem Führungsbereich mit zumindest einem Gleitelement, welches an einem biegesteife Sägeblattkern angeordnet ist und eine Kontaktfläche zur Führung des Sägeblatts in einem Führungsschlitz einer Sägeschablone bereitstellt, und einem distalen, Sägezähne aufweisenden Sägebereich. Das Gleitelement und der Sägeblattkern weisen (jeweils) zueinander unterschiedliche Materialien auf oder bestehen (jeweils) aus zueinander unterschiedlichen Materialen, wobei das Gleitelement einen biokompatiblen und/oder bioresorbierbaren Kunststoff aufweist oder aus diesem besteht. Ferner bildet der Sägeblattkern ein gitter- oder fischgrätenartiges Gerüst, welches eine Anordnung von miteinander verbundenen Stegen oder Streben aufweist und von dem Gleitelement ummantelt ist.

Zusammenfassend besteht die Idee der Erfindung darin, dass ein chirurgisches Sägeblatt mit biokompatiblem Kunststoff bzw. Kunststoffgleitelementen ganz oder teilweise umschlossen wird. Die Lösung kann auf alle oszillierenden Sägeblätter, welche in einer Sägeschablone/ Sägeblock/ Kniesägeschablone geführt oder im Allgemeinen eine Relativbewegung zu vergleichbaren Führungsflächen durchführen, angewandt werden.

Die tribologischen Gegebenheiten wurden im Rahmen der Erfindung durch die Verwendung von Kunststoff und Stahl als Reibpartner anstatt der Interaktion zwischen Stahl und Stahl neugestaltet und optimiert. Das Verbundsägeblatt schützt dabei die Sägeschablone gegen Oberflächenbeschädigung durch Friktion an den Führungsflächen im Sägeschlitz des Sägeblockes. Die Adaption eines mit Kunststoff ganz oder teilweise umschlossenen/besetzten Sägeblattes an die standardmäßig verwendeten Sagittalsägen oder Sägevorrichtungen ist problemlos möglich. Das Sägeblatt gemäß der Erfindung muss mit den gängigsten Führungsspalt- bzw. Sägeblattdicken korrelieren. Es ist unabhängig vom Hersteller in Verbindung mit diversen Sägeblöcken verwendbar. Das heißt, ein biegesteifer (Metall-) Kern oder Sägeblattkern des Sägeblattes ist dabei in seiner Dicke reduziert. Das Verbundsägeblatt erhält erst durch die Kunststoffgleitelemente seine Nenndicke. Die weitverbreitetste, gängige (Nenn-) Sägeblattdicke ist dabei t = 1,27 mm, an welcher sich die Gesamtdicke des Sägeblatts, d.h., eines Sägeblattkerns in Kombination mit dem/n darauf angebrachten Gleitelement/en orientiert bzw. dieser entsprechend dimensioniert ist. Das erfindungsgemäße Sägeblatt ist somit in Kombination mit beliebigen Standard-Sägeschablonen anwendbar oder kann an patientenspezifische Sägeschablonen aus Kunststoff angepasst werden.

Abschließend ist ein biokompatibler Kunststoff wie beispielsweise PEEK oder Polylactide (PLA) oder ABS oder ein vergleichbarer biokompatibler Kunststoff für das Anbringen, insbesondere durch Umspritzen, an dem biegesteifen Sägeblattkerns zu verwenden. Der Einsatz eines resorbierbaren Kunststoffes als Gleitfläche auf dem Verbundsägeblatt ist ferner ebenso vorstellbar.

Die Herstellung des Verbundsägeblatt und das damit verbundene Hinzufügung/Anbringen von Kunststoffgleitelementen an dem Sägeblatt ist über technische Fertigungsverfahren wie Spritzgießen, additive Fertigungsverfahren (z.B. durch Filament- und Granulat-3D-Druck, was auch als Zweikomponentendruck anwendbar ist), Schäumen oder Fügen durch Klebeverbindung vorstellbar. Auch eine formschlüssige Verbindung durch Anklippen, Verkeilen, Einrasten usw. der Kunststoffgleitelemente ist denkbar. Dies ist insbesondere dann von Vorteil, wenn das/die Gleitelement/e austauschbar ist/sind.

Zusammenfassend basiert die der Erfindung zugrunde liegende Idee auf der Erkenntnis, dass durch ein von Kunststoff ganz oder teilweise umschlossenes Verbundsägeblatt durch diesen Werkstoffverbund die Masse des Sägeblattes reduziert wird und/oder Reibung bzw. Abrieb minimiert wird. Oder in anderen Worten ausgedrückt, schützt das Verbundsägeblatt, aufweisend oder bestehend aus einen/m bevorzugt metallischen Sägeblattkern und eine/r teilweisen oder vollständigen Kunststoffummantelung (Kunststoffgleitelemente) die Führungsflächen der Sägeschablonen. Die Folge ist neben der Reduzierung der Abriebpartikel eine Reduzierung der Wärmeentwicklung durch Friktion während der Knieoperation, wodurch die Patentenbelastung weiter herabgesetzt werden kann. Die Ausstattung der Stahlsägeblätter mit Kunststoffgleitelementen ermöglicht eine Reduzierung der schwingenden Masse des Sägeblattes und stellt eine bis dato noch nie da gewesenen Werkstoffkombination der Reibpartner im Einsatzbereich von Kniesägeblättern und dazugehörigen Sägeschablonen/Sägeblöcken dar.

In anderen Worten ausgedrückt, können durch den Verbund eines biegesteifen Sägeblattkerns mit einem Gleitelement aus Kunststoff eine Massenreduktion und somit eine Verminderung der benötigten Akkuleistung beim Sägen sowie ein geringeres Akkugewicht erreicht werden. Darüber hinaus hat ein mit Kunststoff ganz oder teilweise versehenes, insbesondere umspritztes Verbundsägeblatt folgende Vorteile: Die Führungsflächen an der Schablone/dem Sägeblock werden geschützt und unterliegen weniger Korrosion. Die Sägeschablonen/-blöcke erreichen längere Standzeiten bzw. Lebenszyklen. Es werden aufgrund des geringeren Spiels (insbesondere aufgrund geringerer Abnutzung) im Führungsspalt der Schablone genauere Sägeschnitte erzielt. Lärmemission im OP können reduziert werden. Es entsteht aufgrund geringerer Reibung weniger Wärme und somit kann der Wärmeeintrage im Situs-Bereich (d.h., die Wärmebelastung des Patientengewebes) reduziert werden. Eine Reduzierung der Abriebpartikel, insbesondere metallischer Abriebpartikel, wird erreicht. Insbesondere wenn ein bioresorbierbarer Kunststoff für das Gleitelement verwendet wird, werden lediglich biologisch abbaubare Abriebpartikel erzeugt, welche besonders ungefährlich für den Patienten sind. Entsprechend ist es möglich, ein Risiko einer Verursachung/Hervorhebung einer Allergie oder eines allergischen Anfalls des Patienten gegen Bestandteile wie Chrom, Molybdän, Nickel o.Ä., welche in Abriebpartikeln von konventionellen Schablonen und/oder Sägeblättern aus Stahl vorhanden wären, zu verringern und damit verbunden auch das Infektionsrisiko bei der OP direkt zu reduzieren. Die Erfindung ermöglicht es folglich, die Sägeschablone und vor allem den Patienten beim operativen Eingriff zu schützen.

In Versuchen hat sich herausgestellt, dass das erfindungsgemäße Verbundsägeblatt einen sehr guten Sägeschnitt im Eschenholz ermöglicht. Das Verbundsägeblatt, insbesondere die Kunststoffummantelung (Kunststoffgleitelemente), bleibt dabei unbeschädigt. Nach mehreren Sägeschnitten ist keine signifikante Temperaturerhöhung am Verbundsägeblatt oder der Sägeschablone festzustellen. In ersten Versuchen konnte bereits eine Dezimierung der Masse um 50% gegenüber einem vergleichbaren konventionellen Kniesägeblatt nachgewiesen werden.

Für alle später beschriebenen Varianten oder Ausführungsformen des erfindungsgemäßen Verbundsägeblattes steht dessen Anwendung in einer konventionellen Kniesägeschablone/Sägeblock/Sägeschablone im Vordergrund, um viele der vorstehend beschriebenen Vorteile nutzen zu können. Bei allen Varianten wird eine deutliche Reduktion der schwingenden Masse, d.h. eine Reduktion der Sägeblattmasse erreicht.

Vorteilhafte Ausgestaltungen sind Gegenstand der Unteransprüche und werden nachfolgend genauer beschrieben.

Nach einem Aspekt der Erfindung ist es von Vorteil, wenn der Sägebereich gleitelementfrei ausgebildet ist und distal über den Führungsbereich hinausragt. Alternativ oder zusätzlich ist es von Vorteil, wenn der Sägebereich bevorzugt lateral und/oder in einer Dickenrichtung über den Sägeblattkern und das zumindest eine Gleitelement hinausragt. Dadurch kann gewährleistet werden, dass ein Sägeschnitt besonders sauber ausgeführt werden kann, ohne dass ein Risiko einer Beschädigung des zumindest einen Gleitelements durch einen Kontakt mit dem Knochen besteht. Ferner ist dadurch die Fertigung des Gleitelements vereinfacht. In anderen Worten ausgedrückt ist es wichtig, dass der Sägezahnbesatz gegenüber der weiteren Dicke (des Sägeblatts) leicht überstehend ist, um ein Freischneiden des Verbundsägeblattes im Femur- und/oder Tibiaknochen bei der Knochenresektion gewährleisten zu können. Ein Freischneiden kann dabei durch ein Schränken der Sägezähne oder andere aus dem Stand der Technik bekannte Möglichkeiten erfolgen. Ferner kann der Anschlussbereich (Haltebereich) des Sägeblattes dabei frei von Kunststoff gehalten werden.

Nach einer bevorzugten Ausgestaltung der Erfindung ist ein einzelnes, insbesondere durchgängiges, weiter insbesondere lückenfreies, sich über den Führungsbereich erstreckendes Gleitelement vorgesehen. Das Gleitelement kann in diesem Fall plattenförmig auf einer Oberfläche des Sägeblatts angeordnet sein, vorzugsweise derart, dass es den Führungsbereich vollständig bedeckt oder ggf. geringfügig darüber hinausragt, was für eine besonders gleichmäßige Führung dient. Ferner ist es in diesem Fall denkbar, dass das Gleitelement aufklippbar und somit besonders einfach montierbar und ggf. austauschbar gestaltet ist. Alternativ kann das Gleitelement bevorzugt als eine den Führungsbereich vollständig ummantelte Schicht ausgebildet sein. Das heißt, es wird eine rund um den Führungsbereich geschlossene Kunststoffschicht als das Gleitelement bereitgestellt. Dies ist durch z.B. durch Umspritzen oder Beschichten einfach und kostengünstig zu fertigen und erreicht dadurch, dass die Kunststoffschicht in sich geschlossen ist, eine besonders gute Stabilität.

In anderen Worten ausgedrückt, kann es sich bei den vorzugsweise (vollständig) umspritzten Kunststoffgleitelement(en) um eine vollummantelte Ausführung handeln. Das heißt, der biegesteife Metallkern, also das Sägeblatt (der Sägeblattkern), ist von einer Kunststoffschicht umgeben. Ausgenommen davon sind der Sägezahnbesatz (Sägebereich) und der Anschlussbereich (Haltebereich), welcher eine sichere Adaption des Verbundsägeblattes an eine chirurgische Handsäge zulässt.

Ein weiteres Maß (ein weiterer Vorteil der aus Kunststoff ausgebildeten Gleitelemente) sind vielfältige vorsehbare Oberflächengeometrien der Kunststoffgleitflächen (der Oberflächen/Kontaktflächen der Gleitelemente), welche über ein mit Kunststoff versehenes, z.B. umspritztes Kniesägeblatt realisiert werden können. Ein Schlagwort im Zuge dessen ist die Texturierung der aufgebrachten oder umspritzten Kunststoffgleitflächen in Anlehnung an aus der Bionik stammende optimierte tribologischen Eigenschaften. In diesem Zuge ist es insbesondere von Vorteil, das Gleitelement mit einer reibungsreduzierenden Textur zu versehen, insbesondere in Form von Mulden/Näpfchen oder von Schuppen, insbesondere einer schlangenhautartigen Textur. Derartige Texturen können makroskopisch oder mikroskopisch, insbesondere durch Lasern einer Oberfläche des Gleitelements hergestellt sein und die Gleiteigenschaften des Gleitelements wesentlich verbessern. Dadurch wird die Reibung zwischen dem Sägeblatt und dem Führungsschlitz zusätzlich minimiert.

In anderen Worten ausgedrückt, handelt es sich bei den bereitgestellten bzw. umspritzten Kunststoffgleitelementen nach dem vorstehenden Aspekt der Erfindung um eine großflächige Ausführung (d.h. ein platten- oder mantelförmiges Gleitelement) mit einer Näpfchentextur zur Reduzierung der Reibung und der Verbesserung der Gleiteigenschaften. Alternativ oder zusätzlich kann es sich bei den umspritzten Kunststoffgleitelementen um eine großflächige Ausführung mit einer Texturierung in Anlehnung an die Textur einer Schlangenhaut auf Basis von bionischen Erkenntnissen zur Reduzierung der Reibung und der Verbesserung der Gleiteigenschaften handeln. Ferner können verschiedenen Texturen kombiniert werden, z.B. derart, dass an einem proximalen Ende und an einem distalen Ende, welche nur während einer relativ kurzen Zeit während einer Operation innerhalb des Führungsschlitzes geführt sind, eine unterschiedliche Textur vorgesehen ist, als an einer Sägeblattmitte, welche nahezu durchgehend innerhalb des Führungsschlitzes geführt ist und demgemäß anderen Belastungen unterliegt.

Erfindungsgemäss bildet der Sägeblattkern (zumindest im Führungsbereich) ein gitter- oder fischgrätenartiges Gerüst, welches von dem Gleitelement ummantelt ist. Weiter erfindungsgemäss bildet der Sägeblattkern ein Gerüst, das eine Anordnung von miteinander verbundenen Stegen oder Streben enthält oder daraus besteht. Vorzugsweise ist eine Fläche des Gerüsts (der Stege oder Streben, die das Gerüst bilden) im Wesentlichen gleich groß oder kleiner als eine Fläche von Gerüstzwischenräumen des Sägeblatts oder des Führungsbereichs (d.h. von zwischen den Streben oder Stegen liegenden Bereichen). Dadurch kann eine besonders hohe Gewichtsreduktion bei einer gleichzeitig hohen Steifigkeit des Sägeblatts erzielt werden. Ferner kann das Gleitelement z.B. durch Umspritzen an dem Gerüst angebracht werden, wodurch das Gerüst und das Kunststoffmaterial des Gleitelements ineinander eingreifen und ein besonders hoher Halt zwischen diesen erzielbar ist. Das Gerüst ist ferner nicht auf eine Fischgrätenform reduziert. Alternativ ist es z.B. denkbar, zueinander versetzt angeordnete "Gräten" oder Querstreben vorzusehen, ein Gitter, ein leiterförmiges Gerüst oder eine Lochplatte als Sägeblattkern vorzusehen. Im Fall der Lochplatte sind in dem Führungsbereich Durchgangslöcher vorgesehen, deren Anordnung und Durchmesser vorzugsweise derart bemessen sind, dass zwischen den Durchgangslöchern lediglich (schmale) Stege oder Streben stehen bleiben und somit zwischen den Durchgangslöchern das Gerüst gebildet ist. Ein Durchmesser oder eine Fläche der Durchgangslöcher ist vorzugsweise größer als eine Breite der dazwischenliegenden Streben. Ein derartig ausgebildeter Sägeblattkern kann auch in Kombination mit sämtlichen vorstehend und nachstehend beschriebenen Aspekten und Modifikationen der Erfindung verwendet werden. Selbst eine Kombination mit später beschriebenen, einzeln darauf angeordneten, den Sägeblattkern nicht rundum ummantelnden Gleitelementen ist denkbar, wobei dies nicht Teil der Erfindung ist.

In anderen Worten ausgedrückt, wird die Bauform des Verbundsägeblatt nach der Erfindung durch einen gitter- oder fischgrätenartigen (Metall-) Kern ausgedrückt. Dieser fischgrätenartige (Metall-) Kern bildet in Folge dessen das in anderen Ausführungen verwendete Sägeblatt. Dieser fischgrätenartige Metallkern wird von einer Kunststoffschicht umgeben, z.B. umspritzt. Der Sägezahnbesatz und der Anschlussbereich sind wiederum von der Kunststoffschicht freigelassen.

Die im Rahmen dieser Anmeldung beschriebenen Aspekte und Modifikationen der Erfindung betreffen insbesondere ein Sägeblatt, welches ganz oder teilweise zur Reibungsreduzierung in der Sägeschablone mit Kunststoff ummantelt ist und komplett mit einem einteiligen (Stahl-) Kern ausgebildet ist, welcher den Halte-/Kupplungs-, Führungs-/Zwischen und Sägebereich (Sägezähne) aufweist/umfasst. Alternativ, nach einem weiteren bevorzugten Aspekt der Erfindung hat der Sägeblattkern einen distalen, den Sägebereich aufweisenden Teil und einen proximalen, dazu separaten (d.h. nicht stoffeinstückig damit ausgebildet / als ein gesondertes Element / gesondert davon fertigbares Element), den Haltebereich (und vorzugsweise zumindest teilweise den Führungsbereich) aufweisenden Teil, welche (insbesondere ausschließlich) durch das Gleitelement miteinander verbunden oder verbindbar sind. Insbesondere sind der proximale und der distale Teil zueinander beabstandet angeordnet. Eine Lücke zwischen dem distalen und dem proximalen Teil wird insbesondere durch das die Teile verbindende Gleitelement überbrückt. Alternativ können der proximale und der distale Teil (puzzleartig oder kammartig) ineinander eingreifen oder aneinander anliegen/einander kontaktieren, wobei das Gleitelement die beiden Teile (insbesondere die Kontaktstelle zwischen den beiden Teilen) umgibt und diese somit aneinanderhält. Vorzugsweise kann der distale Teil des Sägeblattkerns teilweise den Führungsbereich aufweisen oder können sowohl der distale als auch der proximale Teil jeweils teilweise den Führungsbereich aufweisen, was insbesondere für eine einfachere Verbindung mit dem Gleitelement von Vorteil ist. In anderen Worten ausgedrückt, verbindet das Gleitelement die beiden Teile des Sägeblattkerns, indem es diese ummantelt und eine Kontaktstelle zwischen dem distalen und dem proximalen Teil, an welcher diese aneinander anliegen, überbrückt oder alternativ eine Lücke zwischen dem distalen und dem proximalen Teil überbrückt. Das Verbundsägeblatt kann somit aus zwei oder mehr (separaten) Teilen gebildet sein.

Darüber hinaus ist es besonders bevorzugt, wenn der distale Teil aus einem ersten, insbesondere keramischen Werkstoff gebildet ist und der proximale Teil aus einem zweiten, insbesondere metallischen Werkstoff gebildet ist. Das heißt, die einzelnen Teile können auch verschiedene Werkstoffen aufweisen oder daraus bestehen. Z.B. können ein Hauptteil des Sägeblattkerns aus Stahl und ein Schneidenbereich (Sägebereich) aus Keramik sein, welche durch die Kunststoffummantelung verbunden sind. In diesem Fall ist es besonders vorteilhaft, wenn der distale Teil kurz ist, um einen Einfluss von negativen, durch die Sprödigkeit des keramischen Materials verursachten Eigenschaften gering zu halten. Bevorzugt kann ferner der distale Teil lösbar bzw. austauschbar an dem Gleitelement angebracht oder anbringbar sein, was es ermöglicht, einen beschädigten oder für eine spezifische Anwendungssituation angepassten Sägebereich des Sägeblatts auszutauschen oder zu wechseln. Weiter bevorzugt ist der proximale, vorzugweise metallische Teil deutlich länger als der distale Teil und erstreckt sich nahezu durch den gesamten Führungsbereich, um diesen zu stabilisieren. Dadurch, insbesondere durch die Kombination eines keramischen Sägeblattkerns im Sägezahnbereich und eines metallischen Sägeblattkerns im Halte- und Führungsbereich, können optimale Stabilisierungs- und Schneideigenschaften bereitgestellt werden.

Auch nach den übrigen, im Rahmen dieser Anmeldung beschriebenen Aspekten und Modifikationen der Erfindung kann ein mehrteiliger Sägeblattkern, wie vorstehend beschrieben, gewählt werden, sofern dieser angemessen über das Gleitelement verbindbar ist.

Nach einem weiteren vorteilhaften Aspekt der Erfindung ragt das Gleitelement mit einem Randabschnitt lateral über den Sägeblattkern hinaus. Oder in anderen Worten ausgedrückt, ist eine Überlappung der Kunststoffschicht (des Gleitelements) gegenüber dem (Metall-) Kern im Randbereich vorgesehen. Dies schützt die Sägeschablone beim möglichen Anschlagen/Kollision mit dem oszillierenden Verbundsägeblatt. Dadurch wird die Lebensdauer der Sägeschablone erhöht und Abrieb zusätzlich vermieden. Das Resultat dieser Variante ist ferner ein hohe Biegesteifigkeit im (Metall-) Kern und eine äußere Kunststoffschicht (Gleitelement) mit guten Gleit-und Abriebeigenschaften bezogen auf die Anwendung in einer gängigen Sägeschablone.

Es ist besonders von Vorteil, wenn der Randabschnitt einen weicheren Kunststoff aufweist als ein den Sägeblattkern überlagernder Mittenabschnitt des Gleitelements. Der Mittenabschnitt ist bevorzugt aus einem besonders glatten, abriebfesten Kunststoff gefertigt. Der weiche Kunststoff am Randabschnitt hat bevorzugt gute Dämpfungs- und ggf. Federungseigenschaften. In anderen Worten ausgedrückt, ist diese Bauform des Verbundsägeblatt dadurch gekennzeichnet, dass das Sägeblatt durch das Umschließen mit einer Kunststoffschicht voll ummantelt wird. Der besondere Vorteil liegt darin, dass der Randbereich/-abschnitt im Vergleich zur Basis Kunststoffschicht (des Mittenabschnitts) mit einem weicheren Kunststoffmaterial ausgeführt wird oder daraus besteht. Das Resultat dieser Variante ist eine hohe Biegesteifigkeit im (Metall-) Kern des Verbundsägeblatts und eine äußere Kunststoffschicht mit guten Gleit-und Abriebeigenschaften bezogen auf die Anwendung in einer gängigen Sägeschablone. Besonders ist bei dieser Variante die Verwendung von zwei verschiedenen Kunstoffen (herstellbar z.B. durch einen Zweikomponenten-Spritzguss oder 3D-Druck), nämlich das weichere Kunststoffmaterial im Randbereich/-abschnitt und die grundständige, abriebfeste Kunststoffschicht (des Mittenabschnitts). Die Kombination beider Kunststoffmaterialen bildet letztendlich das Kunststoffgleitelement des Verbundsägeblattes.

Nach einem weiteren bevorzugten Aspekt der Erfindung weist der Randabschnitt zumindest einen sich entlang des Sägeblattkerns erstreckenden Federmechanismus auf, welcher dazu angepasst ist, sich bei einer lateralen Krafteinwirkung elastisch zu verformen. In anderen Worten ausgedrückt, wird, nicht wie vorstehend beschrieben durch die Auswahl des Materials, sondern anhand einer geeigneten Formgebung oder Struktur eine federnde und/oder dämpfende Eigenschaft des Randbereichs erzielt. Obwohl bevorzugt der Federmechanismus als Teil des Gleitelements oder damit verbunden ausgebildet ist, ist es alternativ denkbar, dass der Federmechanismus unmittelbar an einem Rand des Sägeblattkerns z.B. steckbar, angebracht ist.

Ein derartiger Federmechanismus kann auch mit einem wie vorstehend beschrieben aus zwei verschiedenen Kunststoffmaterialien gebildeten Gleitelement, mit einem weicheren Material am Randbereich, kombiniert werden. Ferner können an verschiedenen Teilen des Sägeblatts, welche ggf. unterschiedlichen Anforderungen und Belastungen unterliegen, verschiedene Strukturen und/oder Materialien kombiniert werden. So kann beispielsweise an einem distalen Bereich, welcher beim Sägen besonders stark oszilliert und demgemäß besonders wahrscheinlich und hart mit dem Rand des Führungsschlitzes kollidiert, der bei der lateral wirkenden Kraft besonders gut verformbare und dämpfende Federmechanismus bereitgestellt sein, wohingegen an einem proximalen Bereich, welcher nur wenig wahrscheinlich und voraussichtlich nicht sehr hart mit dem Rand des Führungsschlitzes kollidiert, ein einfacher, aus einem weicheren Kunststoffmaterial gebildeter, über den Sägekern überstehender Gleitelementteil bereitgestellt ist.

In anderen Worten ausgedrückt, zeichnet sich diese Bauform des Verbundsägeblatts dadurch aus, dass das Sägeblatt durch das Umschließen mit einer Kunststoffschicht voll ummantelt wird. Der Unterschied dieser Variante gegenüber der vorstehenden Variante mit dem Randbereich aus einem weicheren Kunststoffmaterial liegt darin, dass der Randbereich im Vergleich zur abriebfesten Kunststoffschicht (des Mittenabschnitts oder der Hauptfläche des Gleitelements), mit federnden Kunststoffelementen ausgeführt wird. Die federnden Kunststoffelemente werden über eine geeignete geometrische Struktur gebildet.

Die geeignete geometrische Struktur kann z.B. aneinandergereihte, lateral nach außen gespannte Bügel aufweisen, welche bei einer lateralen Kraftwirkung elastisch eingedrückt werden können. Alternativ oder zusätzlich ist, insbesondere bei steril vorbereiteten und für eine Einwegverwendung vorgesehenen Sägeblättern, eine gras- oder teppichartige Struktur mit dich aneinander angeordneten, sich nach außen erstreckenden, elastisch verformbaren, ggf. wellenförmig ausgebildeten Stäbchen oder Fasern denkbar. Beispielsweise können die Stäbchen oder Fasern schräg (pfeilartig oder bogenförmig) vom seitlichen Rand des Führungsbereichs/des Sägeblattkerns/des Gleitelements abstehen (d.h., nach außen sowie nach vorne oder nach hinten), um bei einer seitlichen Belastung nach innen zum Sägeblattkern hin einzufedern. Alternativ oder zusätzlich kann eine Schicht in einer Wirrlage angeordneter Fasern vorgesehen sein. Es ist jedoch besonders bevorzugt, dass der Federmechanismus eine leiterförmige, parallellogrammartig verformbare Gitterstruktur aufweist. Leiterförmig ist in diesem Fall derart zu interpretieren, dass sich von dem Gleitelement oder dem Sägeblattkern (jeweils dessen seitlichem Rand) Leitersprossen nach lateral außen erstrecken und an ihren äußeren Enden über einen Leiterholm bzw. eine Verbindungsstange verbunden sind. Als innerer Leiterholm dient in diesem Fall ein (seitlicher) Rand des Gleitelements oder des Sägeblattkerns. Ferner kann die Leiterform auch in Ruhestellung bereits parallellogrammartig vorgeformt sein, um eine bessere Krafteinleitung zu gewährleisten. Diese Struktur ist sehr stabil und ermöglicht dadurch, dass die Gitterstruktur nicht nur lateral nach innen, sondern darüber hinaus in proximaler oder distaler Richtung verformt bzw. verschoben wird, einen besonders langen Federweg und besonders gute Dämpfungseigenschaften. Die Leitersprossen können in regelmäßigen Intervallen entlang des Rands des Führungsbereichs angeordnet sein oder können in engeren oder weiteren Abständen angeordnet sein, beispielsweise um an bestimmten Abschnitten eine härtere bzw. weichere Federung zu erzielen. Gleichermaßen kann die Länge der Sprossen entlang des Führungsbereichs variieren, beispielsweise um an einem distalen Abschnitt einen längeren Federweg bereitzustellen (d.h., vorzugsweise können die Sprossen distal länger ausgebildet sein). Die Federwirkung des Federmechanismus wird aufgrund einer elastischen Verformung der Gitterstruktur bei der lateralen Krafteinwirkung bereitgestellt. Es ist jedoch auch denkbar, Teile des leiterförmigen Gitters gelenkig zu gestalten. Z.B. können die Sprossen am inneren Leiterholm gelenkig angebracht sein (z.B. über eine scharnierartige Steckverbindung), wobei die elastische Rückstellkraft über die elastische Verformung der Verbindungsstelle zwischen dem äußeren Leiterholm und den Sprossen bereitgestellt wird, oder umgekehrt (gelenkartige Verbindung der Sprossen mit dem äußeren Leiterholm und elastische Verformung der Verbindung der Sprossen mit dem inneren Leiterholm).

Diese geometrische Struktur kann durchgängig oder, wie später genauer beschrieben, auch unterbrochen ausgeführt sein. Bei einer seitlichen/lateralen Krafteinwirkung, beispielsweise verursacht durch das Anschlagen an die Sägeschablone, verformt/en sich die federnden Kunststoffelemente bzw. der Federmechanismus in distaler oder proximaler Richtung. Die federnden Kunststoffelemente können dabei aus einem der Basis-Kunststoffschicht (des Mittenabschnitts oder eines Haupt- bzw. Gleitbereichs der Gleitelemente) gleichwertigen Kunststoff, insbesondere stoffeinstückig damit, oder aus einem zweiten alternativen Kunststoff, wie vorstehend beschrieben, hergestellt sein (Stichwort: zwei Komponenten). Das Resultat dieser Variante ist eine hohe Biegesteifigkeit im (Metall-) Kern des Verbundsägeblatts und eine äußere Kunststoffschicht (Gleitelement) mit guten Gleit- und Abriebeigenschaften bezogen auf die Anwendung in einer gängigen Sägeschablone.

Ferner ist es von Vorteil, wenn der Federmechanismus eine Anzahl von entlang des Sägeblattkerns verteilten, insbesondere unabhängig voneinander verformbaren oder verschiebbaren Teil-Federmechanismen/Federabschnitten aufweist. Dies entspricht einer vorstehend genannten, unterbrochenen geometrischen Struktur, was bedeutet, dass unabhängig voneinander verschiebbare/verformbare, federnde Kunststoffelemente bereitgestellt sind. Bei einer solchen unterbrochenen Struktur (des Federmechanismus) können sich diese federnden Kunststoffelemente oder Teil-Federmechanismen unabhängig voneinander verformen und/oder verschieben. Auf diese Weise werden Teile des Federmechanismus, welche nicht mit dem Rand des Führungsschlitzes o.Ä. kollidieren, nicht unnötig verformt und dadurch belastet. Zudem kann eine Abfederung einer lateralen Kraft dadurch weicher erfolgen, als wenn ein durchgehender, entlang des Rands des Gleitelements oder Sägeblattkerns verlaufender Federmechanismus vorgesehen wird.

Nach einer weiteren Ausgestaltung, die nicht Teil der Erfindung ist, kann eine Vielzahl von separaten, insbesondere kreis-/punkt- oder sichelförmigen Gleitelementen über den Führungsbereich verteilt angeordnet sein. In diesem Fall ist vorzugsweise der Sägeblattkern einstückig ausgebildet.

In diesem Fall ist weiter eine Überlappung der angebrachten oder umspritzten Kunststoffgleitelemente am Sägeblatt vorgesehen, um eine solide Führung des Sägeblattes in der Sägeschablone ohne ein Verkippen gewährleisten zu können. Das heißt, um eine sichere Abstützung und Führung in der Sägeschablone zu erreichen, muss ein ausreichender Teil des Führungsabschnitts mit Gleitelementen versehen sein. Am Beispiel der sichelförmigen Gleitelemente (Sicheln) kann dies dadurch erreicht werden, dass die Sicheln in distaler Richtung ineinander geschachtelt oder genested in einer Reihe angeordnet sind und sich mit ihren Sichelspitzen bis in die Nähe des Rands oder bis genau zum Rand des Sägeblattkerns erstrecken. Auf diese Weise kann sowohl ein Verkippen innerhalb des Führungsschlitzes als auch ein Verkanten an einer Kante desselben verhindert werden. Am Beispiel der Kunststoffgleitelemente mit einer geometrische Ausführung in Kreis- oder Punktform können die Gleitelemente vorzugsweise gleichmäßig an den lateralen Rändern des Führungsbereichs verteilt und zu den Punkten oder Kreisen des jeweils gegenüberliegenden Rands versetzt angeordnet sein.

### Figurenbeschreibung

Nachfolgend wird die vorliegende Erfindung anhand bevorzugter Ausführungsformen beschrieben. Diese sind jedoch nur veranschaulichender Natur und sollen den Schutzumfang der vorliegenden Erfindung nicht einschränken. Ferner werden bei der Beschreibung der verschiedenen Ausführungsformen nur deren Unterschiede zueinander genauer beschrieben und werden für gleiche Bauteile dieselben Bezugszeichen verwendet, um redundante Beschreibungen derselben zu vermeiden.
Fig. 1 zeigt eine Sägeanordnung, beispielhaft mit einem Sägeblatt nach einer ersten Ausführungsform der Erfindung.
Fig. 2 zeigt perspektivisch das Sägeblatt nach der ersten Ausführungsform.
Fig. 3 zeigt in einer Draufsicht schematisch ein Sägeblatt nach einer zweiten Ausführungsform.
Fig. 4 zeigt in einer Draufsicht schematisch ein Sägeblatt nach einer dritten Ausführungsform.
Fig. 5 zeigt in einer Draufsicht schematisch ein Sägeblatt nach einer vierten Ausführungsform.
Fig. 6 zeigt in einer Draufsicht schematisch ein Sägeblatt nach einer fünften Ausführungsform.
Fig. 7 zeigt in einer Draufsicht schematisch ein Sägeblatt nach einer sechsten Ausführungsform.
Fig. 8 zeigt perspektivisch schematisch ein Sägeblatt nach einer siebten Ausführungsform.
Fig. 9 zeigt in einer Draufsicht schematisch ein Sägeblatt nach einer achten Ausführungsform.
Fig. 10 zeigt in einer Draufsicht schematisch ein Sägeblatt nach einer neunten Ausführungsform.

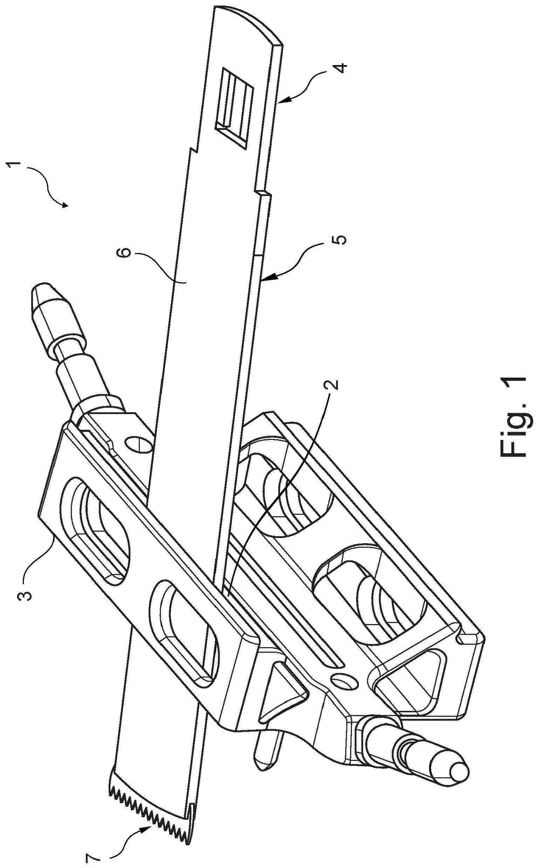

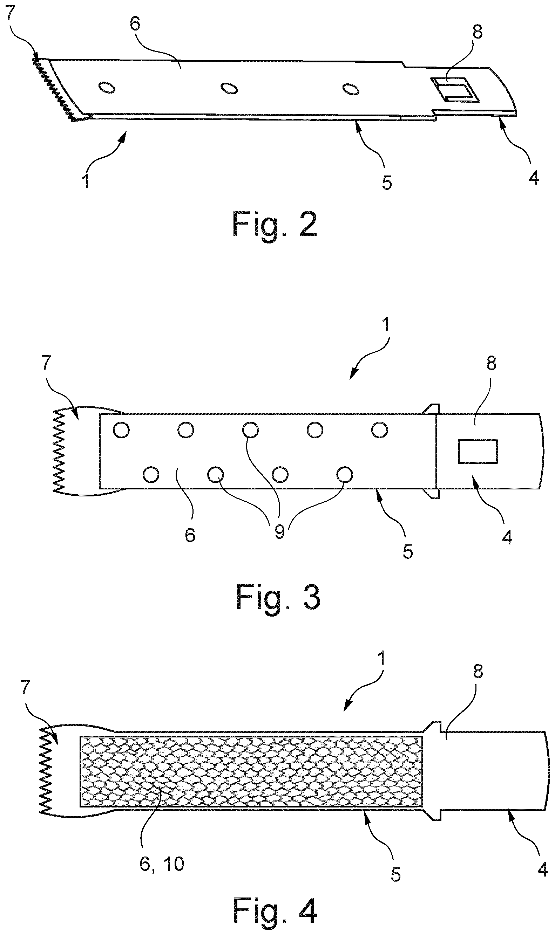

Die in Fig. 1 dargestellte Sägeanordnung zeigt eine Anordnung eines erfindungsgemäßen (Verbund-) Sägeblatts 1, welches in einem Führungsschlitz 2 einer/m standardmäßigen (Knie-) Sägeschablone 3 oder (Knie-) Sägeblock, vorzugsweise aus Metall, insbesondere Stahl, aufgenommen und geführt ist. Während einer Femur- und/oder Tibiaresektion ist die Sägeschablone 3 an einer Führungsstange angebracht, welche z.B. relativ zu einem Patienten, insbesondere dessen Tibia, lagefest angeordnet wird, sodass das Sägeblatt 1 in der Sägeschablone 3 kontrolliert und präzise geführt werden kann. Das in Fig. 1 und nochmals separat in Fig. 2 dargestellte Sägeblatt 1 ist beispielhaft ein Sägeblatt 1 nach einer ersten Ausführungsform der Erfindung, wobei dies durch ein Sägeblatt 1 nach jeder der nachfolgend beschriebenen Ausführungsformen austauschbar ist. Der nachfolgend mit Bezug auf Fig. 1 beschriebene, grundlegende Aufbau ist sämtlichen Ausführungsformen gemein.

Bei dem im wesentlichen plattenförmigen Sägeblatt 1 können funktionsbasiert verschiedene Bereiche unterschieden werden: An einem proximalen Ende des Sägeblatts 1 befindet sich ein Haltebereich 4, welcher dazu dient, das Sägeblatt 1 an einer medizinischen Sägevorrichtung austauschbar anzubringen und hierfür eine vorzugsweise standardisierte Schnittstelle aufweist. Die hier dargestellte Schnittstelle weist beispielhaft eine Eingriffsöffnung ggf. mit Federelementen, Haltekanten und/oder distale Anschläge auf. Von dem Haltebereich 4 erstreckt sich in distaler Richtung ein Führungsbereich 5 des Sägeblatts 1, welcher in der hier dargestellten, einer Verwendung während eines chirurgischen Eingriffs entsprechenden Anordnung durch den Führungsschlitz 2 der Sägeschablone 3 verläuft und darin gleitend geführt ist. An dem Führungsbereich 5 ist ein Gleitelement 6 bereitgestellt, welches eine Kontaktfläche zur gleitenden Führung in dem Führungsschlitz 2 dient. An einem distalen Ende, welches während einer Anwendung dem Patienten zugewandt ist und welches aus der Sägeschablone 3 hervorragt, ist ein Sägebereich 7 bereitgestellt, welcher Sägezähne aufweist und einen Endeffektor bildet. Der Sägebereich 7 sowie vorzugsweise zumindest ein Teil des Haltebereichs 4 sind gleitelementfrei ausgebildet bzw. sind nicht von dem Gleitelement 6 bedeckt. D. h., insbesondere der Sägebereich 7 steht in distaler Richtung sowie (wie besser aus nachfolgenden Figuren hervorgeht) in lateraler Richtung und in Dickenrichtung über den Führungsbereich 5 bzw. das Gleitelement 6 über. Eine Außenkontur des Sägeblatts 1 entspricht im Wesentlichen der eines Standardsägeblatts.

Nach der ersten Ausführungsform weist das in Fig. 2 einzeln dargestellte Sägeblatt 1 einen vorzugweise metallischen, insbesondere einstückigen Sägeblattkern 8 auf, welcher plattenförmigen ist und im Wesentlichen eine Grundform des Sägeblatts 1 vorgibt. Die Grundform ist im Wesentlichen rechteckig. Eine Dicke des Sägeblatts 1 ist geringer als eine Nenndicke eines Standardsägeblatts. Das Gleitelement 6 bildet eine Platte, welche eine Oberseite des Sägeblattkerns 8 insbesondere den Führungsbereich 5 und den Haltebereich 4, nahezu vollständig bedeckt. An einer Unterseite des Sägeblattkerns 8 obgleich in dieser Ansicht nicht zu sehen, ist ein weiteres entsprechendes Gleitelement 6 bereitgestellt, sodass das Sägeblatt 1 in dem Führungsschlitz 2 beidseitig gleitend geführt ist. D. h., der Sägeblattkern 8 ist sandwichartig zwischen den beiden Gleitelementen 6 angeordnet. Insbesondere sind diese Gleitelement 6 bereitgestellt, indem der Sägeblattkern 8 im Führungsbereich 5 umspritzt wird und somit (radial und in Dickenrichtung) vollständig ummantelt ist. Demgemäß ist der Sägeblattkern 8 in Fig. 2 nur am Sägebereich 7 und an einem Teil des Haltebereichs 4 zu sehen, wo er aus den Gleitelementen 6 hervorragt, um die Sägezähne bzw. die Schnittstelle zur Kopplung mit der Sägevorrichtung freizulegen. Gemeinsam bilden diese Komponenten einen Verbund, dessen Dicke einer Nenndicke eines Standardsägeblatts entspricht und welche demgemäß mit einer standardmäßigen Sägeschablone 3 verwendbar ist.

Fig. 3 zeigt schematisch eine zweite Ausführungsform des erfindungsgemäßen Sägeblatts 1. Dieses entspricht im Wesentlichen dem vorstehend bereits beschriebenen Sägeblatt 1, weshalb nachfolgend lediglich auf deren Unterschiede eingegangen wird. In dem Sägeblatt 1 nach der zweiten Ausführungsform weist das Gleitelement 6 eine insbesondere durch Lasern hergestellte Textur oder Oberflächenstruktur in Form von Mulden 9 oder Näpfchen auf, wodurch Gleiteigenschaften des Sägeblatts 1 verbessert und dessen Reibung in dem Führungsschlitz 2 reduzierbar ist. Die Mulden 9 sind dabei gleichmäßig, insbesondere entlang von seitlichen/lateralen Rändern des Gleitelements 6 verteilt und zu den jeweils gegenüberliegenden Mulden 9 versetzt, auf dem Gleitelement 6 verteilt angeordnet. Ferner erstreckt sich das Gleitelement 6 nicht über den Haltebereich 4.

Eine dritte Ausführungsform nach Fig. 4 unterscheidet sich von der zweiten Ausführungsform anhand der reibungsminimieren Textur des Gleitelements 6, welche Schuppen 10 ausbildet, insbesondere schlangenhautartig ausgebildet ist. Eine Größe der durch das Gleitelement 6 bereitgestellten Fläche kann bei den ersten bis dritten Ausführungsformen sowohl in lateraler als auch ein distaler Richtung variieren, sofern zuverlässige, verkippungsfreie Führung des Sägeblatts 1 innerhalb des Führungsschlitzes 2 gewährleistet ist.

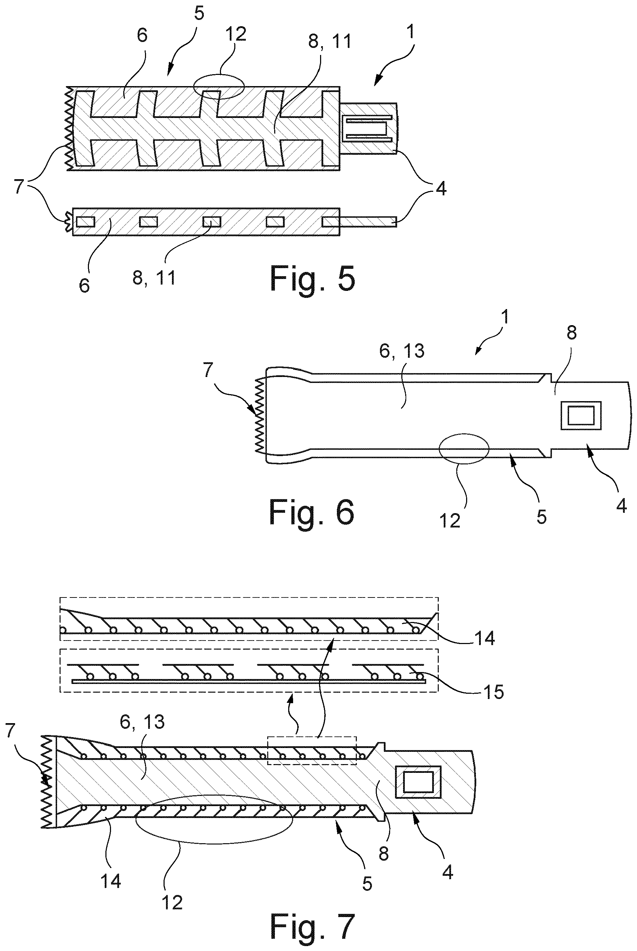

Fig. 5 zeigt schematisch eine vierte Ausführungsform des erfindungsgemäßen Sägeblatts 1 in einer Draufsicht (oben) sowie einer Seitenansicht (unten). Der (einstückige) Sägeblattkern 8 ist fischgrätenartig ausgebildet und stellt ein Gerüst 11 dar, welches mit dem Gleitelement 6 zumindest in lateraler Richtung und in Dickenrichtung im Führungsbereich 5 vollständig umspritzt ist. Ferner ist das Gleitelement 6 in lateraler Richtung breiter als das fischgrätenartige Gerüst 11 und ragt demgemäß seitlich, an einem Randabschnitt 12 des Sägeblatts 1 über das Gerüst 11 (über dessen sich lateral erstreckende Gräten/Arme) hinaus. Dadurch werden das Sägeblatt 1 und die Sägeschablone 3 geschützt, wenn das Sägeblatt 1 während dessen Verwendung oszilliert und lateral an einem Rand des Führungsschlitzes das 2 bzw. der Sägeschablone 3 anschlägt. In der Seitenansicht des gut zu sehen, dass die Sägezähne des Sägebereich 7 in Dickenrichtung über das Gleitelement 6 hervorstehen. Ferner ist schematisch eine Lage des Sägeblattkerns 8 innerhalb des Gleitelements 6, beidseitig von diesem bedeckt, dargestellt.

Fig. 6 zeigt als eine fünfte Ausführungsform eine Adaption des Sägeblatts 1 nach der ersten Ausführungsform, gemäß welcher der rechteckige (einstückige) Sägeblattkern 8 mit einem beidseitig bereitgestellten, flächigen Gleitelement 6 versehen ist. Das Gleitelement 6 gemäß Fig. 6 steht im Unterschied dazu an einem Randabschnitt 12 deutlich über den Sägeblattkern 8 hinaus. Ferner ist das Gleitelement 6 aus zwei verschiedenen Kunstoffen gebildet, sodass ein harter, gute Gleiteigenschaften aufweisender und gegebenenfalls texturierter Mittenabschnitt 13 den Führungsbereich 4 großteils, vorzugsweise vollständig, bedeckt und der Randabschnitt 12 aus einem weicheren Kunststoff zur Dämpfung und/oder Federung von Kollisionen des Sägeblatts 1 mit dem Führungsschlitz 2 gebildet ist oder besteht.

Die in Fig. 7 dargestellte sechste Ausführungsform ermöglicht es, Dämpfungs- und/oder Federeigenschaften eines Randabschnitts 12 im Hinblick auf laterale Krafteinwirkungen wie z.B. bei Kollisionen deutlich zu verbessern. Das Sägeblatt 1 nach dieser Ausführungsform ist in Fig. 7, unten, in Draufsicht dargestellt und hat beidseitig an seinen Randabschnitten 12 entlang des Führungsbereichs 5 jeweils einen Federmechanismus 14 oder der jeweilige Randabschnitt 12 ist als ein Federmechanismus 14 ausgebildet. Der Federmechanismus 14 wird durch eine leiterartige, aus Kunststoff gefertigte Gitterstruktur gebildet, welche sich bei einem Auftreten einer lateralen Kraft, z.B. durch eine Kollision mit dem Rand des Führungsschlitzes 2, parallellogrammartig verformt bzw. verschiebt. Einer der Leiterholme wird dabei durch den Rand des Sägeblattkerns 8 oder des Gleitelements 6 gebildet und der gegenüberliegende Leiterholm bildet einen Außenrand des Gesamtsägeblatts 1. Um Beschädigungen zu vermeiden und die parallellogrammartige Verformung besser einzuleiten, ist bevorzugt die leiterartige Gitterstruktur bereits im unbelasteten Ruhezustand parallellogrammartig bzw. schräg ausgebildet. Die Gitterstruktur kann einstückig, insbesondere stoffeinstückig, mit dem Gleitelement 6 ausgebildet sein, wobei die parallellogrammartige Verformung basierend auf einer elastischen Verformung, insbesondere an Ecken oder Schnittstellen von Leitersprossen mit den Leiterholmen, des Gitters erfolgt, um durch die elastische Rückstellkraft des Gittermaterials den Federmechanismus 14 bereitzustellen. Alternativ oder zusätzlich können Elemente des Gitters scharnierartig verbunden sein, inbesondere nach innen gewandte Enden der Leitersprossen, welche an dem Gleitelement 6 oder einem Rand des (einstückigen) Sägeblattkerns 8 federnd angelenkt sein können. Ggf. können die Leitersprossen innenseitig angelenkt sein und außenseitig elastisch verformbar mit dem Leiterrand verbunden sein.

In Fig. 7, oben, sind in Detailansichten A und B des Randabschnitts 12 Modifikationen des Federmechanismus 14 dargestellt. Detailansicht A zeigt einen Federmechanismus 14, welcher als eine durchgängig entlang des gesamten Führungsbereichs 5 verlaufende, leiterartige Gitterstruktur gebildet ist. Detailansicht B zeigt eine Modifikation, gemäß welcher der Federmechanismus 14 eine leiterartige Gitterstruktur aufweist, die entlang des Führungsbereichs 5, insbesondere in gleichmäßigen Intervallen, unterteilt ist, um eine Anzahl von unabhängig zueinander, wie vorstehend beschrieben, parallellogrammartig verformbaren oder verschiebbaren Teil-Federmechanismen 15 bereitzustellen.

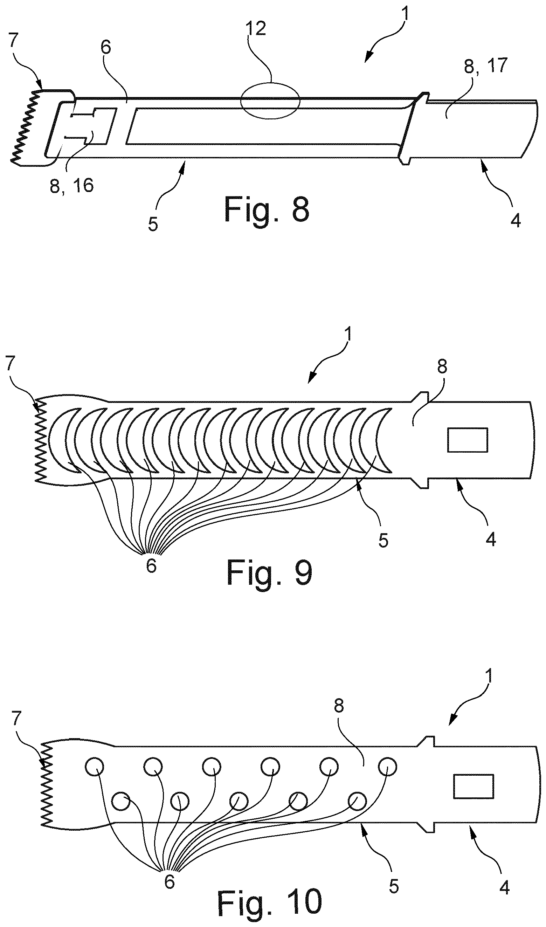

Fig. 8 zeigt eine siebte Ausführungsform des erfindungsgemäßen Sägeblatts 1. Dieses Sägeblatt 1 hat einen zweiteiligen Sägeblattkern 8, wobei ein distaler Teil 16 des Sägeblattkerns 8 den Sägebereich 7 sowie teilweise den Führungsbereich 5 aufweist und ein proximaler Teil 17 des Sägeblattkerns 8 den Haltebereich 5 sowie teilweise den Führungsbereich 5 aufweist. Der distale Teil 16 und der proximale Teil 17 des Sägeblattkerns 8 sind durch das sich über den Führungsbereich erstreckendende, diesen vollständig ummantelnde und eine Lücke zwischen dem distalen und dem proximalen Teil 16, 17 überbrückenden Gleitelement 6 verbunden. Der der Teil des Führungsbereichs 5, welcher dem distalen Teil 16 des Sägeblattkerns 8 zugeordnet ist, ist kurz und dient hauptsächlich als Verbindungselement zur ggf. lösbaren/austauschbaren Verbindung des distalen Teils 16 mit dem Gleitelement 6. Der Teil des Führungsbereichs 5, welcher dem proximalen Teil 17 des Sägeblattkerns 8 zugeordnet ist, ist lang und erstreckt sich nahezu durch den gesamten Führungsbereich 5, um diesen zu stabilisieren. Vorzugsweise ist der proximale Teil 17 des Sägeblattkerns 8 aus einem Metall gefertigt und ist der distale Teil 16 des Sägeblattkerns 8 aus einer Keramik gefertigt, um optimale Stabilisierungs- und Schneideigenschaften bereitzustellen. Auch in dieser Ausführungsform ummantelt das Gleitelement 6 den Sägeblattkern 8 derart, dass dieser an einem Randbereich 12 lateral übersteht.

Fig. 9 zeigt ein Sägeblatt 1 nach einer achten Ausführungsform, der nicht Teil der Erfindung ist. Der Führungsbereich 5 weist eine Vielzahl von separaten, sichelförmigen Gleitelementen 6 auf, welche auf einem einstückigen Sägeblattkern 8 verteilt angeordnet sind. Die durch die Gleitelemente 6 gebildeten Sicheln sind entlang einer sich von proximal nach distal erstreckenden Mittellinie des Führungsbereichs 5 spiegelsymmetrisch angeordnet, sodass sie miteinander fluchten und jeweils in Richtung distal konvex gewölbt sind. Die einzelnen Sicheln sind dabei ausreichend nahe zueinander angeordnet, dass eine sichere, verkippungsfreie Führung innerhalb des Führungsschlitzes 2 der Sägeschablone 3 gewährleistet ist. Vorzugsweise überlappen sich die Sicheln teilweise, d.h., sind ineinander genestet oder derart angeordnet, dass eine konvexe Wölbung der Sicheln jeweils in einer konkaven Mulde oder Gegenseite der nebenliegenden Sichel aufgenommen ist. Auf diese Weise kann sowohl ein Verkippen innerhalb des Führungsschlitzes 2 als auch ein Verkanten an einer Kante desselben verhindert werden.

Fig. 10 zeigt ein Sägeblatt 1 nach einer neunten Ausführungsform, der auch nicht Teil der Erfindung ist, und welches sich von dem der achten Ausführungsform lediglich anhand der Form und Anordnung der einzelnen Gleitelemente 6 unterscheidet. Genauer ausgedrückt, sind die Gleitelemente 6 punkt- oder kreisförmig und auf dem Sägeblattkern 8 gleichmäßig über den Führungsbereich 5 verteilt angeordnet. Insbesondere sind die durch die Gleitelemente 6 gebildeten Punkte oder Kreise gleichmäßig an den lateralen Rändern des Führungsbereichs verteilt und zu den Punkten oder Kreisen des jeweils gegenüberliegenden Rands versetzt angeordnet.

### Referenzzeichenliste

- 1: Sägeblatt
- 2: Führungsschlitz
- 3: (Knie-) Sägeschablone/-block
- 4: Haltebereich
- 5: Führungsbereich
- 6: Gleitelement
- 7: Sägebereich
- 8: Sägeblattkern
- 9: Mulden (Textur)
- 10: Schuppen (Textur)
- 11: Gerüst
- 12: Randabschnitt
- 13: Mittenabschnitt
- 14: Federmechanismus
- 15: Teil-Federmechanismus
- 16: distaler Teil des Sägeblattkerns
- 17: proximaler Teil des Sägeblattkerns

## Patentansprüche

1. Sägeblatt (1) für eine medizinische Knochensäge, insbesondere für eine Femur- und/oder Tibiaresektion, mit
einem proximalen Haltebereich (4), welcher zur Kopplung an einer Sägevorrichtung dient,
einem Führungsbereich (5) mit zumindest einem Gleitelement (6), welches an einem biegesteife Sägeblattkern (8) angeordnet ist und eine Kontaktfläche zur Führung des Sägeblatts (1) in einem Führungsschlitz (2) einer Sägeschablone (3) bereitstellt, und
einem distalen, Sägezähne aufweisenden Sägebereich (7),
wobei
das Gleitelement (6) und der Sägeblattkern (8) zueinander unterschiedliche Materialien aufweisen oder aus zueinander unterschiedlichen Materialien bestehen, wobei das Gleitelement (6) einen biokompatiblen und/oder bioresorbierbaren Kunststoff aufweist oder aus diesem besteht, **dadurch gekennzeichnet, dass**
der Sägeblattkern (8) ein gitter- oder fischgrätenartiges Gerüst (11) bildet, welches eine Anordnung von miteinander verbundenen Stegen oder Streben aufweist und von dem Gleitelement (6) ummantelt ist.

2. Sägeblatt (1) nach Anspruch 1, **dadurch gekennzeichnet, dass** der Sägebereich (7) gleitelementfrei ausgebildet ist und distal über den Führungsbereich (5) hinausragt.

3. Sägeblatt (1) nach einem der Ansprüche 1 und 2, **dadurch gekennzeichnet, dass** der Sägebereich (7) lateral und/oder in einer Dickenrichtung über den Sägeblattkern (8) und das zumindest eine Gleitelement (6) hinausragt.

4. Sägeblatt (1) nach einem der Ansprüche 1 bis 3, **dadurch gekennzeichnet, dass** ein einzelnes, insbesondere durchgängiges, sich über den Führungsbereich (5) erstreckendes Gleitelement (6) vorgesehen ist.

5. Sägeblatt (1) nach einem der Ansprüche 1 bis 4, **dadurch gekennzeichnet, dass** das Gleitelement (6) als eine den Führungsbereich (5) vollständig ummantelte Schicht ausgebildet ist.

6. Sägeblatt (1) nach einem der Ansprüche 4 und 5, **dadurch gekennzeichnet, dass** das Gleitelement (6) eine reibungsreduzierende Textur aufweist, insbesondere in Form von Mulden (9) oder von Schuppen (10).

7. Sägeblatt (1) nach Anspruch 1, **dadurch gekennzeichnet, dass** eine Fläche des Gerüsts (11) im Wesentlichen gleich groß oder kleiner als eine Fläche von Gerüstzwischenräumen ist.

8. Sägeblatt (1) nach einem der Ansprüche 4 bis 6, **dadurch gekennzeichnet, dass** der Sägeblattkern (8) einen den Sägebereich (7) aufweisenden distalen Teil (16) und einen dazu separaten, den Haltebereich (4) und vorzugsweise zumindest teilweise den Führungsbereich (5) aufweisenden proximalen Teil (17) ausbildet, welche durch das Gleitelement (6) miteinander verbunden oder verbindbar sind.

9. Sägeblatt (1) nach Anspruch 8, **dadurch gekennzeichnet, dass** der distale Teil (16) aus einem ersten, insbesondere keramischen Werkstoff gebildet ist und der proximale Teil (17) aus einem zweiten, insbesondere metallischen Werkstoff gebildet ist.

10. Sägeblatt (1) nach einem der Ansprüche 1 bis 9, **dadurch gekennzeichnet, dass** das Gleitelement (6) mit einem Randabschnitt (12) lateral über den Sägeblattkern (8) hinausragt.

11. Sägeblatt (1) nach Anspruch 10, **dadurch gekennzeichnet, dass** der Randabschnitt (12) einen weicheren Kunststoff aufweist als ein den Sägeblattkern (8) überlagernder Mittenabschnitt des Gleitelements (6).

12. Sägeblatt (1) nach Anspruch 10, **dadurch gekennzeichnet, dass** der Randabschnitt (12) zumindest einen sich entlang des Sägeblattkerns (8) erstreckenden Federmechanismus (14) aufweist, welcher dazu angepasst ist, sich bei einer lateralen Krafteinwirkung elastisch zu verformen.

13. Sägeblatt (1) nach Anspruch 12, **dadurch gekennzeichnet, dass** der Federmechanismus (14) eine leiterförmige, parallellogrammartig verformbare Gitterstruktur aufweist.

14. Sägeblatt (1) nach Anspruch 12 oder 13, **dadurch gekennzeichnet, dass** der Federmechanismus (14) eine Anzahl von entlang des Sägeblattkerns (8) verteilten, insbesondere unabhängig voneinander verformbaren oder verschiebbaren Teil-Federmechanismen (15) aufweist.

## Claims

1. A saw blade (1) for a medical bone saw, in particular for a femur and/or tibia resection, with
a proximal holding area (4), which serves for coupling to a sawing device,
a guide area (5) with at least one sliding element (6), which is arranged on a non-bendable saw blade core (8) and provides a contact surface for guiding the saw blade (1) in a guide slot (2) of a saw template (3), and
a distal sawing area (7) having saw teeth,
wherein the sliding element (6) and the saw blade core (8) have different materials or consist of different materials with respect to each other, wherein the sliding element (6) has or consists of a biocompatible and/or bioresorbable plastic, **characterized in that**
the saw blade core (8) forms a lattice-like or herringbone-like framework (11), which has an arrangement of interconnected webs or struts and is encased by the sliding element (6).

2. The saw blade (1) according to claim 1, **characterized in that** the sawing area (7) is free of sliding elements and projects distally beyond the guide area (5).

3. The saw blade (1) according to one of claims 1 and 2, **characterized in that** the sawing region (7) projects laterally and/or in a thickness direction beyond the saw blade core (8) and the at least one sliding element (6).

4. The saw blade (1) according to one of claims 1 to 3, **characterized in that** a single, in particular continuous, sliding element (6) extending over the guide area (5) is provided.

5. The saw blade (1) according to one of claims 1 to 4, **characterized in that** the sliding element (6) is configured as a layer completely encasing the guide region (5).

6. The saw blade (1) according to one of claims 4 and 5, **characterized in that** the sliding element (6) has a friction-reducing texture, in particular in the form of troughs (9) or scales (10).

7. The saw blade (1) according to claim 1, **characterized in that** an area of the framework (11) is substantially equal to or smaller than an area of framework gaps.

8. The saw blade (1) according to one of claims 4 to 6, **characterized in that** the saw blade core (8) forms a distal part (16) having the sawing region (7) and a separate proximal part (17) having the holding region (4) and preferably at least partially the guide region (5), which are connected or connectable to each other by the sliding element (6).

9. The saw blade (1) according to claim 8, **characterized in that** the distal part (16) is formed from a first, in particular ceramic, material and the proximal part (17) is formed from a second, in particular metallic, material.

10. The saw blade (1) according to one of claims 1 to 9, **characterized in that** the sliding element (6) projects laterally beyond the saw blade core (8) with an edge section (12).

11. The saw blade (1) according to claim 10, **characterized in that** the edge section (12) has a softer plastic than a central section of the sliding element (6) overlying the saw blade core (8).

12. The saw blade (1) according to claim 10, **characterized in that** the edge section (12) has at least one spring mechanism (14) extending along the saw blade core (8), which is adapted to deform elastically when a lateral force is applied.

13. The saw blade (1) according to claim 12, **characterized in that** the spring mechanism (14) has a ladder-shaped, parallelogram-like deformable grid structure.

14. The saw blade (1) according to claim 12 or 13, **characterized in that** the spring mechanism (14) has a number of partial spring mechanisms (15) which are distributed along the saw blade core (8) and in particular can be deformed or displaced independently of each other.

## Revendications

1. Lame de scie (1) pour une scie à os médicale, en particulier pour une résection du fémur et/ou du tibia, avec
une zone de retenue proximale (4) qui sert à l'accouplement à un dispositif de scie,
une zone de guidage (5) avec au moins un élément de glissement (6) qui est disposé sur un noyau de lame de scie (8) résistant à la flexion et fournit une surface de contact pour le guidage de la lame de scie (1) dans une fente de guidage (2) d'un gabarit de scie (3), et
une zone de scie distale (7) présentant des dents de scie,
dans laquelle l'élément de glissement (6) et le noyau de lame de scie (8) présentent des matériaux différents l'un de l'autre ou sont constitués de matériaux différents l'un de l'autre, dans laquelle l'élément de glissement (6) présente une matière plastique biocompatible et/ou biorésorbable ou est constitué de celle-ci, **caractérisée en ce que**
le noyau de lame de scie (8) forme une armature (11) en forme de grille ou d'arête de poisson qui présente un agencement de nervures ou d'entretoises connectées entre elles et est enveloppée par l'élément de glissement (6).

2. Lame de scie (1) selon la revendication 1, **caractérisée en ce que** la zone de scie (7) est conçue sans élément de glissement et fait saillie distalement de la zone de guidage (5).

3. Lame de scie (1) selon l'une quelconque des revendications 1 et 2, **caractérisée en ce que** la zone de scie (7) fait saillie latéralement et/ou dans une direction d'épaisseur au-delà du noyau de lame de scie (8) et de l'au moins un élément de glissement (6).

4. Lame de scie (1) selon l'une quelconque des revendications 1 à 3, **caractérisée en ce qu'**un élément de glissement (6) individuel, en particulier continu, s'étendant sur la zone de guidage (5) est prévu.

5. Lame de scie (1) selon l'une quelconque des revendications 1 à 4, **caractérisée en ce que** l'élément de glissement (6) est conçu en tant que couche enveloppant entièrement la zone de guidage (5).

6. Lame de scie (1) selon l'une quelconque des revendications 4 et 5, **caractérisée en ce que** l'élément de glissement (6) présente une texture réduisant le frottement, en particulier sous forme de creux (9) ou d'écailles (10).

7. Lame de scie (1) selon la revendication 1, **caractérisée en ce qu'**une surface de l'armature (11) est sensiblement égale ou inférieure à une surface d'espaces intermédiaires d'armature.

8. Lame de scie (1) selon l'une quelconque des revendications 4 à 6, **caractérisée en ce que** le noyau de lame de scie (8) forme une partie distale (16) présentant la zone de sciage (7) et une partie proximale (17) séparée de celle-ci, présentant la zone de retenue (4) et de préférence au moins partiellement la zone de guidage (5), lesquelles sont connectées ou peuvent être connectées entre elles par l'élément de glissement (6).

9. Lame de scie (1) selon la revendication 8, **caractérisée en ce que** la partie distale (16) est formée d'un premier matériau, en particulier céramique, et la partie proximale (17) est formée d'un second matériau, en particulier métallique.

10. Lame de scie (1) selon l'une quelconque des revendications 1 à 9, **caractérisée en ce que** l'élément de glissement (6) fait saillie latéralement par une section de bord (12) au-delà du noyau de lame de scie (8).

11. Lame de scie (1) selon la revendication 10, **caractérisée en ce que** la section de bord (12) présente une matière plastique plus souple qu'une section centrale de l'élément de glissement (6) recouvrant le noyau de lame de scie (8).

12. Lame de scie (1) selon la revendication 10, **caractérisée en ce que** la section de bord (12) présente au moins un mécanisme à ressort (14) s'étendant le long du noyau de lame de scie (8) qui est adapté pour se déformer élastiquement lorsqu'une force latérale est appliquée.

13. Lame de scie (1) selon la revendication 12, **caractérisée en ce que** le mécanisme à ressort (14) présente une structure de grille en forme d'échelle déformable à la manière d'un parallélogramme.

14. Lame de scie (1) selon la revendication 12 ou 13, **caractérisée en ce que** le mécanisme à ressort (14) présente un certain nombre de mécanismes à ressort partiels (15), en particulier déformables ou déplaçables indépendamment les uns des autres, répartis le long du noyau de lame de scie (8).
